# EUROPEAN PATENT SPECIFICATION

(11) **EP 3 248 583 B1**
(45) Date of publication and mention of the grant of the patent: **12.12.2018**
(21) Application number: 17173172.2
(22) Date of filing: 29.05.2017
(51) Int. Cl.: A61H 39/00, A61N 1/02, A61N 1/04, A61N 1/36

(54) **MERIDIAN POINT ACTIVATION THERAPY APPARATUS**
MERIDIANPUNKTAKTIVIERUNGSTHERAPIEVORRICHTUNG
APPAREIL DE THÉRAPIE PAR ACTIVATION DE POINT DE MÉRIDIEN

(30) Priority: 27.05.2016 HK 16106071
(43) Date of publication of application: 29.11.2017
(73) Proprietor: Lee, Kam Yin, Tsim Sha Tsui, Kowloon (HK)
(72) Inventor: LEE, Kam Yin, Tsim Sha Tsui, Kowloon (HK); LAU, Kwun Leung, Tsim Sha Tsui, Kowloon (HK); HUNG, Fuk Man, Tsim Sha Tsui, Kowloon (HK)
(74) Representative: Becker Kurig Straus

(56) References cited:
- EP-A1- 0 173 419
- EP-A1- 2 780 073
- WO-A1-2015/187712

## Description

### TECHNICAL FIELD

The present application relates to the field of medical care equipment and, in particular, to a meridian point activation therapy apparatus.

### BACKGROUND

Meridian point activation therapy is based on the principle of Chinese acupuncture and moxibustion for meridians and acupoints, as well as the research results of modern biomedicine. According to the diseases needed for treatment, the meridians and acupoints on a human body are divided into different meridian point combinations. Modern electronic pulse technology is used to stimulate appropriate meridian point combination and nerve points, thereby promoting human blood circulation and enhancing metabolism. It has an excellent effect on adjusting the function of human body's immune system, improving blood quality, comprehensive clearance of the body's meridian pathway, healing and strengthening of the body, prevention of obesity, weight loss, shaping body lines, beauty, anti-aging, and maintenance of health.

### SUMMARY

In a meridian point activation therapy, the intensity of the pulse signal that can be accepted at an initial stage of the therapy is usually small. At this time, the penetration of pulse signal is weak. A patient's acceptance of the intensity can be improved gradually as the therapy time moves on. After reaching a certain intensity and rate, the pulse signal penetration will be significantly improved with good therapeutic effect.

In the course of studying the present therapy apparatus, the inventor has found that mild changes in the intensity of the pulse signal at the initial stage of therapy can directly affect the therapeutic effect. During the therapy, the control of pulse signal intensity will make the patient more likely to accept the increase of intensity, and become faster in reaching a higher level. Hence, the patient can accept a higher maximum intensity. It also helps to shorten the entire therapy process and improve the therapeutic effect.

Meridian point activation therapy apparatus currently existing on the market uses manual adjustment of a potentiometer to adjust the pulse signal intensity. Specifically, a patient is adjusting the potentiometer, while he or she is feeling whether the pulse signal intensity is appropriate. Manual adjustment of potentiometer cannot easily quantify the change of the pulse signal. Therefore, if a user wants to make the initial pulse signal to change mildly, the user needs to accurately control the adjustment of the potentiometer in order to fine-tune the pulse signal. Usually, only users with experience and skills can cope with it. This reduces user's experience, and limits the application of the meridian point activation therapy.

Thus, there is a need for a new type of meridian point activation therapy apparatus to improve the convenience of adjusting the pulse signal parameters.

An object of the present application is to provide a meridian point activation therapy apparatus including an input unit, a control unit, a pulse generator, an electrode, and a display unit.

The input unit may include a first select button and a second select button. The first select button is provided for selection of an intensity change rate by a user, and the second select button is provided for selection of an intensity stable value by the user. The input unit generates a first input signal based on the intensity change rate selected by the user, and generates a second input signal based on the intensity stable value selected by the user.

The control unit may be connected to the input unit and configured to determine the intensity change rate selected by the user based on the first input signal, determine the intensity stable value selected by the user based on the second input signal, send an excitation signal to the pulse generator, and control a change of the excitation signal based on the intensity change rate selected by the user and the intensity stable value selected by the user.

The pulse generator may be connected to the control unit and configured to generate a pulse signal based on the excitation signal. The intensity of the pulse signal changes based on the intensity change rate selected by the user, and remains unchanged until the strength stable value selected by the user is reached.

The electrode may be connected to the pulse generator and configured to output the pulse signal.

The display unit may be connected to the control unit for displaying the intensity change rate and/or the intensity stable value.

By means of the above-mentioned technical proposal, the meridian point activation therapy apparatus of the present application is more advantageous in controlling mild change of the pulse signal intensity, improving the speed and scope of patient's stimulation, shortening the time of the whole therapy process, and making the patient to have better therapeutic effect. The meridian point activation therapy apparatus of the present application only requires setting of two to three parameters at the initial stage, so that the pulse signal can be changed gradually without the need to manually control the pulse signal change so that it is more convenient during the course of the therapy. It is beneficial to the promotion of the application of meridian point activation therapy. WO2015187712 discloses a peripheral nerve stimulator suitable for meridian point activation therapy, with an input unit comprising two select buttons, control unit, pulse generator, electrode, whereby the input unit generates a user selectable intensity change rate and a final intensity stable value.

Although the meridian point activation therapy apparatus is shown and described with respect to certain embodiments, it is obvious that equivalents and modifications will occur to others skilled in the art upon the reading and understanding of the specification. The meridian point activation therapy apparatus in the present application includes all such equivalents and modifications, and is limited only by the scope of the claims.

### BRIEF DESCRIPTION OF THE DRAWINGS

In order to more clearly illustrate the embodiments of the apparatus of the present application or the technical solutions in the prior art, the following drawings, which are intended to be used in the description of the embodiments, are briefly described. It will be apparent that the drawings in the following description are illustrative only. Without making any creative effort, a person skilled in the art may derive other drawings from the drawings in the present application, wherein:
Figure 1 is a block diagram showing an embodiment of the meridian point activation therapy apparatus of the present application.
Figure 2 is a block diagram showing another embodiment of the meridian point activation therapy apparatus of the present application.
Figure 3 is a schematic comparison of the curative effects of the meridian point activation therapy apparatus of the present application and the currently existing meridian point activation therapy apparatus on the same patient.

### DETAILED DESCRIPTION OF EMBODIMENTS

Reference will now be made in detail to a preferred embodiment of the meridian point activation therapy apparatus, examples of which are also provided in the following description. Exemplary embodiments of the meridian point activation therapy apparatus are described in detail, although it will be apparent to those skilled in the relevant art that some features that are not particularly important to an understanding of the meridian point activation therapy apparatus may not be shown for the sake of clarity.

Furthermore, it should be understood that the meridian point activation therapy apparatus is not limited to the precise embodiments described below and that various changes and modifications thereof may be effected by one skilled in the art without departing from the scope of the protection. For example, elements and/or features of different illustrative embodiments may be combined with each other and/or substituted for each other within the scope of this disclosure and appended claims.

In addition, improvements and modifications which may become apparent to persons of ordinary skill in the art after reading this disclosure, the drawings, and the appended claims are deemed within the scope of the protection.

It should be noted that throughout the specification and claims herein, when one element is said to be "coupled" or "connected" to another, this does not necessarily mean that one element is fastened, secured, or otherwise attached to another element. Instead, the term "coupled" or "connected" means that one element is either connected directly or indirectly to another element or is in mechanical or electrical communication with another element.

The technical solution provided by the embodiments of the apparatus of the present application will be described clearly and completely below. It is apparent that the described embodiments are only some embodiments of the apparatus and are not intended to be exhaustive. All other embodiments obtained by an ordinary technical person skilled in the art, without making any creative effort, are within the scope of protection of the apparatus of the present application.

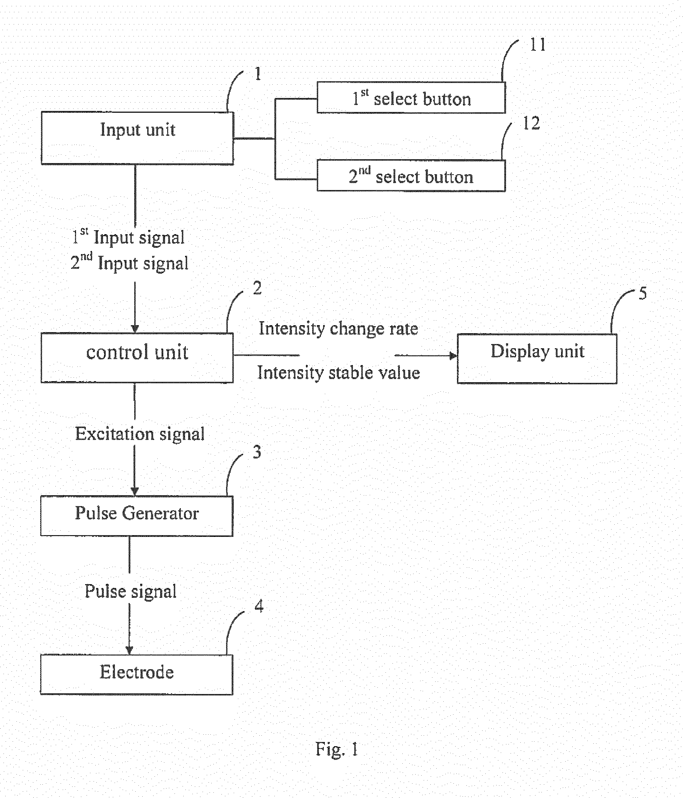

The present application discloses a meridian point activation therapy apparatus. As shown in Fig. 1, the meridian point activation therapy apparatus may include an input unit 1, a control unit 2, a pulse generator 3, an electrode 4, and a display unit 5.

The input unit 1 may include a first select button 11 and a second select button 12. The first select button 11 can be provided for selection of an intensity change rate by a user, and the second select button 12 can be provided for selection of an intensity stable value by the user. The input unit 1 can generate a first input signal based on the intensity change rate selected by the user, and generate a second input signal based on the intensity stable value selected by the user.

The control unit 2 can be connected to the input unit 1 and configured to determine the intensity change rate selected by the user based on the first input signal, determine the intensity stable value selected by the user based on the second input signal, send an excitation signal to the pulse generator 3, and control a change of the excitation signal based on the intensity change rate selected by the user and the intensity stable value selected by the user.

The pulse generator 3 can be connected to the control unit 2 and configured to generate a pulse signal based on the excitation signal. The intensity of the pulse signal changes based on the intensity change rate selected by the user, and remains unchanged until the strength stable value selected by the user is reached.

The electrode 4 can be connected to the pulse generator 3 and configured to output the pulse signal.

The display unit 5 can be connected to the control unit 2 for displaying the intensity change rate and/or the intensity stable value.

The meridian point activation therapy apparatus of the present application is described below. Specifically, the input unit 1 can provide a user with the capability of selecting an intensity change rate by a first select button 11, and the user is also provided with the capability of selecting an intensity stable value by a second select button 12.

In a specific embodiment, the meridian point activation therapy apparatus of the present application can provide a plurality of levels of intensity change rate for the user to select from. The intensity change rate means the amount of change in the intensity of pulse signal applied to a patient's body per unit time. The patient may select a certain level based on his or her own physical ability. It should be noted that the term "pulse signal intensity" means the amplitude of the pulse signal.

Similarly, the meridian point activation therapy apparatus of the present application can provide a plurality of levels of intensity stable value for the user to select from. The term "intensity stable value" means the maximum intensity of the pulse signal applied to the patient's body. The patient may select a level according to his or her own physical ability.

The input unit 1 may generate a first input signal that matches the intensity change rate selected by the user, and the control unit 2 can determine the intensity change rate selected by the user by analyzing the first input signal, and control uniform change of an excitation signal sent to the pulse generator 3, to thereby control uniform change of the pulse signal generated by the pulse generator 3 according to the intensity change rate selected by the user.

Correspondingly, the input unit 1 may generate a second input signal that matches the intensity stable value selected by the user, and the control unit 2 can determine the intensity stable value selected by the user by analyzing the second input signal, and control the change of the excitation signal sent to the pulse generator 3, to thereby control the pulse signal generated by the pulse generator 3 and maintain pulse signal until the intensity stable value selected by the user is reached.

The pulse signal generated by the pulse generator 3 is conducted through the electrode 4 to the patient's body surface, such as a meridian point. The number of electrode 4 may be one or more. For example, a plurality of electrodes 4 may be attached to different meridian points of the patient's body respectively, so that the resulting pulse signals can stimulate these meridian points. The electrode 4 may be linear in shape, or in sheet form, or in any other appropriate form.

Two sets of data can be displayed by the display unit 5 in order to facilitate the patient to confirm the selected intensity change rate and/or the intensity stable value. In one embodiment, the display unit 5 may use a general-purpose 7-segment LED display circuit.

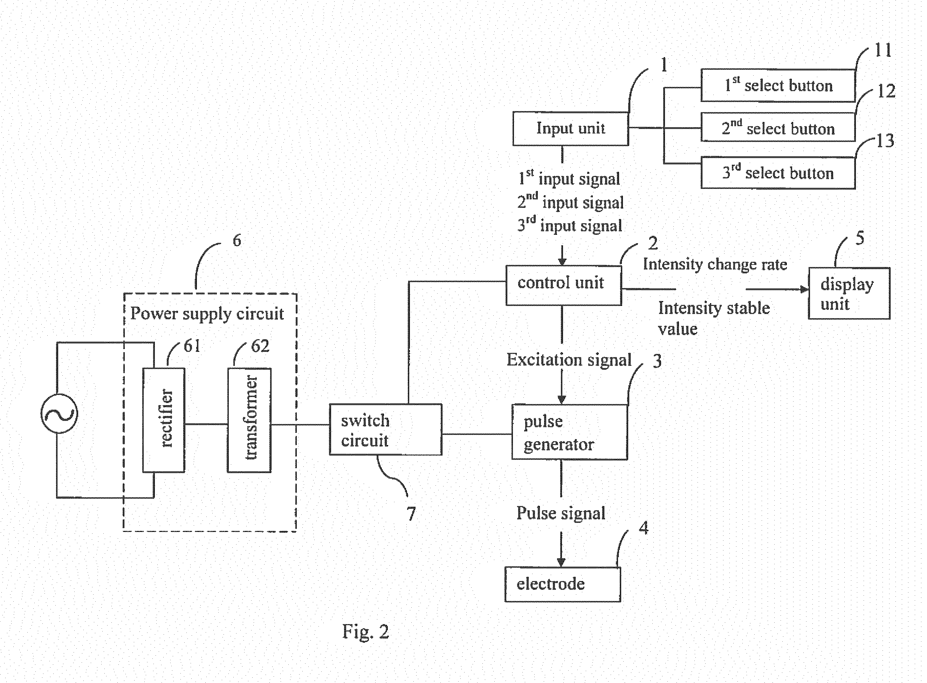

As shown in Fig. 2, the meridian point activation therapy apparatus of the present application may further include a switch circuit 7 connected to the control unit 2 and the pulse generator 3, and a power supply circuit 6 connected to the switch circuit 7.

The input unit 1 may further include a third select button 13 provided for setting a meridian point activation therapy time by the user. The input unit 1 can generate a third input signal based on the meridian point activation therapy time set by the user, and the control unit 2 can be further configured to determine the meridian point activation therapy time set by the user based on the third input signal.

The power supply circuit 6 may include a rectifier 61 and a transformer 62. An input end of the rectifier 61 can be connected to an alternating current and an output end of the rectifier 61 can be connected to the transformer 62 for converting the alternating current to direct current having a constant voltage. An output end of the transformer 62 may be connected to the switch circuit 7 for converting the voltage output by the rectifier 61 to a rated working voltage of the pulse generator 3, and an output end of the switch circuit 7 can be connected to the pulse generator 3.

The control unit 2 may be further configured to determine that when an operating time of the pulse generator 3 has not reached the meridian point activation therapy time set by the user, then control closing of the switch circuit 7; and determine that when the operation time of the pulse generator 3 reaches the meridian point activation therapy time set by the user, then control disconnection of the switch circuit 7. In one embodiment, the control unit 2 may use the countdown function of a timer to determine whether the operation time of the pulse generator 3 has reached the meridian point activation therapy time set by the user.

When the switch circuit 7 is closed, the direct current output from the transformer 62 can be supplied to the pulse generator 3. When the switch circuit 7 is disconnected, the pulse generator 3 is stopped.

Alternatively, the user may also stop the operation of the entire meridian point activation therapy apparatus by disconnecting the alternating current from the power supply circuit 6.

By using the meridian point activation therapy apparatus of the present application, the user only needs to set the intensity change rate, the intensity stable value, and the meridian point activation therapy time at the beginning. The control unit 2 can control operation of the pulse generator 3 according to these three parameters set by the user, so that the pulse signal applied to the patient's body surface can be changed according to the intensity change rate set by the user (i.e., the amount of change of intensity of the pulse signal per unit time is equal to the intensity change rate set by the user). It remains unchanged until after reaching the intensity stable value set by the user, and eventually ends when the meridian point activation therapy time arrives.

In contrast to the use of a potentiometer to adjust the change of pulse signal in the currently existing meridian point activation therapy apparatus, the meridian point activation therapy apparatus of the present application on one hand is more advantageous in controlling mild change of the pulse signal intensity, enhancing the patient's acceptance to the rate and scope of the change in stimulation, shortening the time of the entire therapy process, so that the patient can obtain a better therapeutic effect. On the other hand, the meridian point activation therapy apparatus of the present application only requires setting of two to three parameters at the beginning in order to achieve gradual change of the pulse signal. There is no need to manually control the pulse signal changing during the therapy process. Thus, it is more convenient, and is beneficial to promote the application of meridian point activation therapy.

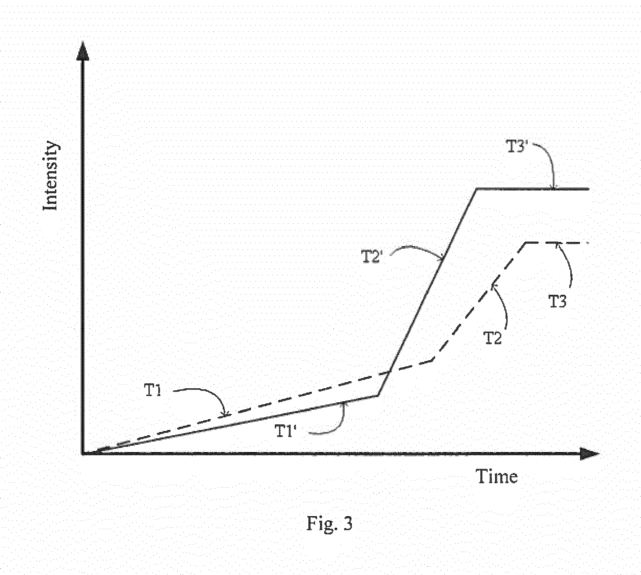

Fig. 3 shows the results of the meridian point activation therapy for the same patient using the meridian point activation therapy apparatus of the present application and the currently existing meridian point activation therapy apparatus (i.e. using potentiometer to adjust the change of pulse signal), wherein the horizontal ordinate indicates the total time for the therapy, and the vertical ordinate indicates the pulse signal intensity.

The two line segments corresponding to T1 and T1' respectively represent the initial period of time of the therapy using the currently existing meridian point activation therapy apparatus and the meridian point activation therapy apparatus of the present application. During the initial period of time, the pulse signal intensity is mildly changed. Hence, the slope of the two line segments corresponding to T1 and T1' is smaller.

The two line segments corresponding to T2 and T2' respectively represent the middle period of time of the therapy using the currently existing meridian point activation therapy apparatus and the meridian point activation therapy apparatus of the present application. During the middle period of time, the change of the pulse signal intensity is faster. Hence, the slope of the two line segments corresponding to T2 and T2' is larger.

The two line segments corresponding to T3 and T3' respectively represent the final period of time of the therapy using the currently existing meridian point activation therapy apparatus and the meridian point activation therapy apparatus of the present application. During the final period of time, the pulse signal intensity is maintained at the intensity stable value.

By comparing T1 and T1', it can be seen that using the meridian point activation therapy apparatus of the present application for the therapy, the initial period of time for the therapy is shortened, and the therapy time is shortened on the premise of achieving the same therapeutic effect.

By comparing T2 and T2', it can be seen that using the meridian point activation therapy apparatus of the present application for the therapy, the intensity acceptable by the patient is increased greater and faster, and the therapy time is shortened on the premise of achieving the same therapeutic effect.

By comparing T3 and T3', it can be seen that using of the meridian point activation therapy apparatus of the present application for the therapy, the patient can finally accept a higher intensity of pulse signal (i.e., greater intensity stable value), the penetration of the pulse signal is significantly improved, and therapeutic effect is also significantly improved.

The foregoing is a detailed description of the objects, technical solutions and advantages of the meridian point activation therapy apparatus. It is to be understood that the foregoing description is merely illustrative of the specific embodiments of the apparatus and is not intended to be limiting. It should be noted that, upon reading the above disclosure, a person skilled in the art can make various other changes, improvements, or modifications without departing from the scope of the appended claims.

Those of skill would further appreciate that the various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the appended claims.

The various illustrative logical blocks, modules, and circuits described in connection with the disclosure herein can be implemented or performed with a general-purpose processor, a digital signal processor, an application specific integrated circuit (ASIC), a field programmable gate array or other programmable logic device, discrete gate or transistor logic, discrete hardware components, or any combination thereof designed to perform the functions described herein. A general-purpose processor can be a microprocessor, but in the alternative, the processor can be any conventional processor, controller, microcontroller, or state machine. A processor can also be implemented as a combination of computing devices, e.g., a combination of a digital signal processor and a microprocessor, a plurality of microprocessors, one or more microprocessors in conjunction with a digital signal processor core, or any other such configuration.

The operations of a method or algorithm described in connection with the disclosure herein can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of storage medium known in the art. An example storage medium is coupled to the processor such that the processor can read information from, and write information to, the storage medium. In the alternative, the storage medium can be integral to the processor. The processor and the storage medium can reside in an ASIC. The ASIC can reside in a user terminal. In the alternative, the processor and the storage medium can reside as discrete components in a user terminal.

In one or more example designs, the functions described can be implemented in hardware, software, firmware, or any combination thereof. If implemented in software, the functions can be stored on or transmitted over as one or more instructions or code on a non-transitory computer-readable medium. Non-transitory computer-readable media includes both computer storage media and communication media including any medium that facilitates transfer of a computer program from one place to another. A storage media can be any available media that can be accessed by a general purpose or special purpose computer. By way of example, and not limitation, such computer-readable media can include RAM, ROM, EEPROM, CD-ROM or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor. Disk and disc, as used herein, includes compact disc, laser disc, optical disc, digital versatile disc (DVD), floppy disk and blue ray disc where disks usually reproduce data magnetically, while discs reproduce data optically with lasers. Combinations of the above should also be included within the scope of non-transitory computer-readable media.

## Claims

1. A meridian point activation therapy apparatus, **characterized in** comprising an input unit (1), a control unit (2), a pulse generator (3), and an electrode (4);
the input unit (1) comprising a first select button (11) and a second select button (12), the first select button (11) provided for selection of an intensity change rate by a user, the second select button (12) provided for selection of an intensity stable value by the user, wherein the input unit (1) generates a first input signal based on the intensity change rate selected by the user, and generates a second input signal based on the intensity stable value selected by the user;
the control unit (2) connected to the input unit (1) and configured to determine the intensity change rate selected by the user based on the first input signal, determine the intensity stable value selected by the user based on the second input signal, send an excitation signal to the pulse generator (3), and control a change of the excitation signal based on the intensity change rate selected by the user and the intensity stable value selected by the user;
the pulse generator (3) connected to the control unit (2) and configured to generate a pulse signal based on the excitation signal, wherein an intensity of the pulse signal changes based on the intensity change rate selected by the user, and said change rate remains unchanged until the strength stable value selected by the user is reached; and
the electrode (4) connected to the pulse generator (3) and configured to output the pulse signal;
the input unit (1) further comprises a third select button (13) provided for setting a meridian point activation therapy time by the user, wherein the input unit (1) generates a third input signal based on the meridian point activation therapy time set by the user, and the control unit (2) is further configured to determine the meridian point activation therapy time set by the user based on the third input signal;
the control unit (2) controls operation of the pulse generator (3) according to the intensity change rate, the intensity stable value, and the meridian point activation therapy time set by the user at the beginning so that the pulse signal can be changed according to the intensity change rate set by the user, said change rate remains unchanged until after reaching the intensity stable value set by the user, and eventually ends when the meridian point activation therapy time set by the user arrives.

2. The meridian point activation therapy apparatus according to claim 1, **characterized in** further comprising a switch circuit (7) connected to the control unit (2) and the pulse generator (3).

3. The meridian point activation therapy apparatus according to claim 2, **characterized in** further comprising a power supply circuit (6) connected to the switch circuit (7).

4. The meridian point activation therapy apparatus according to claim 3, **characterized in that** the power supply circuit (6) comprises a rectifier (61) and a transformer (62), wherein an input end of the rectifier (61) is connected to an alternating current and an output end of the rectifier (61) is connected to the transformer (62) for converting the alternating current to direct current having a constant voltage, and an output end of the transformer (62) is connected to the switch circuit (7) for converting the voltage output by the rectifier (61) to a rated working voltage of the pulse generator (3), and an output end of the switch circuit (7) is connected to the pulse generator (3).

5. The meridian point activation therapy apparatus according to claim 4, **characterized in** the control unit (2) is further configured to
determine that when an operating time of the pulse generator (3) has not reached the meridian point activation therapy time set by the user, then control closing of the switch circuit (7);
determine that when the operation time of the pulse generator (3) reaches the meridian point activation therapy time set by the user, then control disconnection of the switch circuit (7); and
when the switch circuit (7) is closed, the direct current output from the transformer (62) is supplied to the pulse generator (3).

6. The meridian point activation therapy apparatus according to claim 1, **characterized in that** the first select button (11) provides a plurality of levels of the intensity change rate for selection by the user.

7. The meridian point activation therapy apparatus according to claim 1, **characterized in that** the second select button (12) provides a plurality of levels of the intensity stable value for selection by the user.

8. The meridian point activation therapy apparatus according to claim 1, **characterized in** further comprising a display unit (5) connected to the control unit (2).

9. The meridian point activation therapy apparatus according to claim 8, **characterized in that** the display unit (5) displays the intensity change rate.

10. The meridian point activation therapy apparatus according to claim 8, **characterized in that** the display unit (5) displays the intensity stable value.

11. The meridian point activation therapy apparatus according to any one of claims 8 to 10, **characterized in that** the display unit (5) comprises a 7-segment LED display circuit.

12. The meridian point activation therapy apparatus according to claim 1, **characterized in that** the number of the electrode (4) is one or more.

13. The meridian point activation therapy apparatus according to claim 1, **characterized in that** the electrode (4) is linear in shape.

14. The meridian point activation therapy apparatus according to claim 1, **characterized in that** the electrode (4) is in the form of a sheet.

## Patentansprüche

1. Meridianpunkt-Aktivierungstherapievorrichtung, **dadurch gekennzeichnet, dass** sie eine Eingabeeinheit (1), eine Steuereinheit (2), einen Impulserzeuger (3) und eine Elektrode (4) umfasst,
wobei die Eingabeeinheit (1) eine erste Auswahltaste (11) und eine zweite Auswahltaste (12) umfasst, wobei die erste Auswahltaste (11) zum Auswählen einer Intensitätsänderungsrate durch einen Benutzer und die zweite Auswahltaste (12) zum Auswählen eines stabilen Intensitätswerts durch den Benutzer vorgesehen ist, wobei die Eingabeeinheit (1) auf der Grundlage der vom Benutzer ausgewählten Intensitätsänderungsrate ein erstes Eingangssignal und auf der Grundlage des vom Benutzer ausgewählten stabilen Intensitätswerts ein zweites Eingangssignal erzeugt,
wobei die Steuereinheit (2) mit der Eingabeeinheit (1) verbunden und so konfiguriert ist, dass sie die vom Benutzer ausgewählte Intensitätsänderungsrate auf der Grundlage des ersten Eingangssignals bestimmt, den vom Benutzer ausgewählten stabilen Intensitätswert auf der Grundlage des zweiten Eingangssignals bestimmt, ein Anregungssignal zu dem Impulserzeuger (3) sendet und auf der Grundlage der vom Benutzer ausgewählten Intensitätsänderungsrate und des vom Benutzer ausgewählten stabilen Intensitätswerts eine Änderung des Anregungssignals steuert,
wobei der Impulserzeuger (3) mit der Steuereinheit (2) verbunden und so konfiguriert ist, dass er auf der Grundlage des Anregungssignals ein Impulssignal erzeugt, wobei sich die Intensität des Impulssignals auf der Grundlage der vom Benutzer ausgewählten Intensitätsänderungsrate ändert und die Änderungsrate unverändert bleibt, bis der vom Benutzer ausgewählte stabile Stärkewert erreicht ist, und
wobei die Elektrode (4) mit dem Impulserzeuger (3) verbunden und so konfiguriert ist, dass sie das Impulssignal ausgibt,
wobei die Eingabeeinheit (1) ferner eine dritte Auswahltaste (13) umfasst, die zum Einstellen einer Meridianpunkt-Aktivierungstherapiedauer durch den Benutzer vorgesehen ist, wobei die Eingabeeinheit (1) auf der Grundlage der vom Benutzer eingestellten Meridianpunkt-Aktivierungstherapiedauer ein drittes Eingangssignal erzeugt und die Steuereinheit (2) ferner so konfiguriert ist, dass sie die vom Benutzer eingestellte Meridianpunkt-Aktivierungstherapiedauer auf der Grundlage des dritten Eingangssignals bestimmt,
wobei die Steuereinheit (2) den Betrieb des Impulserzeugers (3) der Intensitätsänderungsrate, dem stabilen Intensitätswert und der Meridianpunkt-Aktivierungstherapiedauer entsprechend steuert, die zu Beginn vom Benutzer eingestellt werden, so dass das Impulssignal der vom Benutzer eingestellten Intensitätsänderungsrate entsprechend geändert werden kann, wobei die Änderungsrate unverändert bleibt, bis der vom Benutzer eingestellte stabile Intensitätswert erreicht worden ist, und schließlich endet, wenn die vom Benutzer eingestellte Meridianpunkt-Aktivierungstherapiedauer abgelaufen ist.

2. Meridianpunkt-Aktivierungstherapievorrichtung nach Anspruch 1, **dadurch gekennzeichnet, dass** sie ferner einen Schalterstromkreis (7) umfasst, der mit der Steuereinheit (2) und dem Impulserzeuger (3) verbunden ist.

3. Meridianpunkt-Aktivierungstherapievorrichtung nach Anspruch 2, **dadurch gekennzeichnet, dass** sie ferner einen Versorgungsstromkreis (6) umfasst, der mit dem Schalterstromkreis (7) verbunden ist.

4. Meridianpunkt-Aktivierungstherapievorrichtung nach Anspruch 3, **dadurch gekennzeichnet, dass** der Versorgungsstromkreis (6) einen Gleichrichter (61) und einen Transformator (62) umfasst, wobei ein Eingangsende des Gleichrichters (61) an einen Wechselstrom und ein Ausgangsende des Gleichrichters (61) zum Umwandeln des Wechselstroms in Gleichstrom mit konstanter Spannung an den Transformator (62) angeschlossen ist und ein Ausgangsende des Transformators (62) zum Umwandeln der vom Gleichrichter (61) abgegebenen Spannung in eine Nennarbeitsspannung des Impulserzeugers (3) an den Schalterstromkreis (7) und ein Ausgangsende des Schalterstromkreises (7) an den Impulserzeuger (3) angeschlossen ist.

5. Meridianpunkt-Aktivierungstherapievorrichtung nach Anspruch 4, **dadurch gekennzeichnet, dass** die Steuereinheit (2) ferner so konfiguriert ist, dass sie:
bestimmt, dass sie dann das Schließen des Schalterstromkreises (7) steuert, wenn eine Betriebsdauer des Impulserzeugers (3) noch nicht die vom Benutzer eingestellte Meridianpunkt-Aktivierungstherapiedauer erreicht hat,
bestimmt, dass sie dann das Trennen des Schalterstromkreises (7) steuert, wenn die Betriebsdauer des Impulserzeugers (3) die vom Benutzer eingestellte Meridianpunkt-Aktivierungstherapiedauer erreicht, und,
wenn der Schalterstromkreis (7) geschlossen ist, der von dem Transformator (62) abgegebene Gleichstrom zum Impulserzeuger (3) geleitet wird.

6. Meridianpunkt-Aktivierungstherapievorrichtung nach Anspruch 1, **dadurch gekennzeichnet, dass** die erste Auswahltaste (11) mehrere Stufen für die Intensitätsänderungsrate zur Auswahl durch den Benutzer bereitstellt.

7. Meridianpunkt-Aktivierungstherapievorrichtung nach Anspruch 1, **dadurch gekennzeichnet, dass** die zweite Auswahltaste (12) mehrere Stufen für den stabilen Intensitätswert zur Auswahl durch den Benutzer bereitstellt.

8. Meridianpunkt-Aktivierungstherapievorrichtung nach Anspruch 1, **dadurch gekennzeichnet, dass** sie ferner eine Anzeigeeinheit (5) umfasst, die mit der Steuereinheit (2) verbunden ist.

9. Meridianpunkt-Aktivierungstherapievorrichtung nach Anspruch 8, **dadurch gekennzeichnet, dass** die Anzeigeeinheit (5) die Intensitätsänderungsrate anzeigt.

10. Meridianpunkt-Aktivierungstherapievorrichtung nach Anspruch 8, **dadurch gekennzeichnet, dass** die Anzeigeeinheit (5) den stabilen Intensitätswert anzeigt.

11. Meridianpunkt-Aktivierungstherapievorrichtung nach einem der Ansprüche 8 bis 10, **dadurch gekennzeichnet, dass** die Anzeigeeinheit (5) einen 7-Segment-LED-Anzeigestromkreis umfasst.

12. Meridianpunkt-Aktivierungstherapievorrichtung nach Anspruch 1, **dadurch gekennzeichnet, dass** die Anzahl der Elektrode (4) eine oder mehr beträgt.

13. Meridianpunkt-Aktivierungstherapievorrichtung nach Anspruch 1, **dadurch gekennzeichnet, dass** die Elektrode (4) eine lineare Form aufweist.

14. Meridianpunkt-Aktivierungstherapievorrichtung nach Anspruch 1, **dadurch gekennzeichnet, dass** die Elektrode (4) die Form einer Platte aufweist.

## Revendications

1. Appareil de thérapie par activation de point méridien, **caractérisé en ce que** celui-ci comprend une unité de saisie (1), une unité de commande (2), un générateur d'impulsions (3) et une électrode (4) ;
l'unité de saisie (1) comprenant un premier bouton de sélection (11) et un deuxième bouton de sélection (12), le premier bouton de sélection (11) étant conçu pour sélectionner une vitesse de changement d'intensité par un utilisateur, le deuxième bouton de sélection (12) étant conçu pour sélectionnée une valeur stable d'intensité par l'utilisateur, l'unité de saisie (1) générant un premier signal d'entrée basé sur la vitesse de changement d'intensité sélectionnée par l'utilisateur, et générant un deuxième signal d'entrée basé sur la valeur stable d'intensité sélectionnée par l'utilisateur,
l'unité de commande (2) étant connectée à l'unité de saisie (1) et configurée pour déterminer la vitesse de changement d'intensité sélectionnée par l'utilisateur sur la base du premier signal d'entrée, pour déterminer la valeur stable d'intensité sélectionnée par l'utilisateur sur la base du deuxième signal d'entrée, pour envoyer un signal d'excitation au générateur d'impulsions (3), et pour commander un changement du signal d'excitation sur la base de la vitesse de changement d'intensité sélectionnée par l'utilisateur et de la valeur stable d'intensité sélectionnée par l'utilisateur ;
le générateur d'impulsions (3) étant connecté à l'unité de commande (2) et configuré pour générer un signal d'impulsion basé sur le signal d'excitation, où une intensité du signal d'impulsion varie en fonction de la vitesse de changement d'intensité sélectionnée par l'utilisateur, et ladite vitesse de changement reste inchangée jusqu'à ce que la valeur stable de puissance sélectionnée par l'utilisateur soit atteinte ; et
l'électrode (4) étant connectée au générateur d'impulsions (3) et configurée pour émettre le signal d'impulsion ;
l'unité de saisie (1) comprenant en outre un troisième bouton de sélection (13) conçu pour le réglage d'une durée de thérapie par activation de point méridien par l'utilisateur, l'unité de saisie (1) générant un troisième signal d'entrée sur la base de la durée de thérapie par activation de point méridien réglée par l'utilisateur, et l'unité de commande (2) étant en outre configurée pour déterminer la durée de thérapie par activation de point méridien réglée par l'utilisateur sur la base du troisième signal d'entrée ;
l'unité de commande (2) commandant le fonctionnement du générateur d'impulsions (3) selon la vitesse de changement d'intensité, la valeur stable d'intensité et la durée de thérapie par activation de point méridien réglée par l'utilisateur au début, de manière à ce que le signal d'impulsion puisse être modifié en fonction de la vitesse de changement d'intensité réglée par l'utilisateur, ladite vitesse de changement restant inchangée jusqu'à ce que la valeur stable d'intensité réglée par l'utilisateur soit atteinte, et se terminant éventuellement lorsque la durée de thérapie par activation de point méridien réglée par l'utilisateur est écoulée.

2. Appareil de thérapie par activation de point méridien selon la revendication 1, **caractérisé en ce que** celui-ci comprend en outre un circuit de commutation (7) connecté à l'unité de commande (2) et au générateur d'impulsions (3).

3. Appareil de thérapie par activation de point méridien selon la revendication 2, **caractérisé en ce que** celui-ci comprend en outre un circuit d'alimentation électrique (6) connecté au circuit de commutation (7).

4. Appareil de thérapie par activation de point méridien selon la revendication 3, **caractérisé en ce que** le circuit d'alimentation électrique (6) comprend un redresseur (61) et un transformateur (62), une extrémité d'entrée du redresseur (61) étant connectée à un courant alternatif et une extrémité de sortie du redresseur (61) étant connectée au transformateur (62) pour convertir le courant alternatif en un courant direct présentant une tension constante, et une extrémité de sortie du transformateur (62) étant connectée au circuit de commutation (7) pour convertir la tension fournie par le redresseur (61) en une tension de service nominale du générateur d'impulsions (3), et une extrémité de sortie du circuit de commutation (7) étant connectée au générateur d'impulsions (3).

5. Appareil de thérapie par activation de point méridien selon la revendication 4, **caractérisé en ce que** l'unité de commande (2) est en outre configurée pour :
déterminer que lorsqu'une durée de fonctionnement du générateur d'impulsions (3) n'a pas atteint la durée de thérapie par activation de point méridien réglée par l'utilisateur, le circuit de commutation (7) est commandé pour être fermé,
déterminer que lorsque la durée de fonctionnement du générateur d'impulsions (3) atteint la durée de thérapie par activation de point méridien réglée par l'utilisateur, le circuit de commutation (7) est commandé pour être déconnecté ; et
lorsque le circuit de commutation (7) est fermé, le courant direct fourni depuis le transformateur (62) est fourni au générateur d'impulsions (3).

6. Appareil de thérapie par activation de point méridien selon la revendication 1, **caractérisé en ce que** le premier bouton de sélection (11) fournit une pluralité de niveaux de la vitesse de changement d'intensité pour la sélection par l'utilisateur.

7. Appareil de thérapie par activation de point méridien selon la revendication 1, **caractérisé en ce que** le deuxième bouton de sélection (12) fournit une pluralité de niveaux de la valeur stable d'intensité pour la sélection par l'utilisateur.

8. Appareil de thérapie par activation de point méridien selon la revendication 1, **caractérisé en ce que** celui-ci comprend en outre une unité d'affichage (5) connectée à l'unité de commande (2).

9. Appareil de thérapie par activation de point méridien selon la revendication 8, **caractérisé en ce que** l'unité d'affichage (5) affiche la vitesse de changement d'intensité.

10. Appareil de thérapie par activation de point méridien selon la revendication 8, **caractérisé en ce que** l'unité d'affichage (5) affiche la valeur stable d'intensité.

11. Appareil de thérapie par activation de point méridien selon l'une quelconque des revendications 8 à 10, **caractérisé en ce que** l'unité d'affichage (5) comprend un circuit d'affichage DEL à 7 segments.

12. Appareil de thérapie par activation de point méridien selon la revendication 1, **caractérisé en ce que** le nombre d'électrodes (4) est d'un ou plus.

13. Appareil de thérapie par activation de point méridien selon la revendication 1, **caractérisé en ce que** l'électrode (4) présente une forme linéaire.

14. Appareil de thérapie par activation de point méridien selon la revendication 1, **caractérisé en ce que** l'électrode (4) se présente sous la forme d'une feuille.
